(19) Europäisches Patentamt
European Patent Office
Office européen des brevets

(11) **EP 2 908 716 B1**

(12) **EUROPEAN PATENT SPECIFICATION**

(45) Date of publication and mention of the grant of the patent:
**26.04.2017 Bulletin 2017/17**

(21) Application number: **12809371.3**

(22) Date of filing: **04.09.2012**

(51) Int Cl.:
*A61B 5/00* *(2006.01)*   *A61B 8/08* *(2006.01)*
*A61B 5/05* *(2006.01)*

(86) International application number:
**PCT/TR2012/000136**

(87) International publication number:
**WO 2014/039017 (13.03.2014 Gazette 2014/11)**

(54) **HYBRID MECHANICAL-ELECTROMAGNETIC IMAGING METHOD AND THE SYSTEM THEREOF**

MECHANISCH-ELEKTROMAGNETISCHES HYBRIDES BILDGEBUNGSVERFAHREN UND SYSTEM DAFÜR

PROCÉDÉ D'IMAGERIE MÉCANIQUE-ÉLECTROMAGNÉTIQUE HYBRIDE ET SYSTÈME DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.08.2015 Bulletin 2015/35**

(73) Proprietors:
• **Gencer, Nevzat Guneri**
  **06800 Ankara (TR)**
• **Top, Can Baris**
  **06730 Ankara (TR)**

(72) Inventors:
• **Gencer, Nevzat Guneri**
  **06800 Ankara (TR)**

• **Top, Can Baris**
  **06730 Ankara (TR)**

(74) Representative: **Karaahmet, Erdogan**
**Yalciner Patent and Consulting Ltd.**
**Tunus Cad. 85/3-4**
**Kavaklidere**
**Cankaya**
**06680 Ankara (TR)**

(56) References cited:
**US-A- 5 099 848       US-A- 6 026 173**
**US-A1- 2009 281 422**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### Field of the Invention

The invention is defined by the claims and is related to an imaging system in which a small volume inside an object is vibrated using ultrasound waves, and microwave signals are transmitted to the object and scattered signals are received before and during the vibration. The Doppler component of the received signal - which is due to vibration - is used to form images that reveal the combined electrical and elastic properties of the object.

### State of the Art

Medical imaging systems are being used for diagnostic purposes to get information about tissues. There are several different methods and systems for diagnosis of different kind of diseases and different parts of body. Diagnosis of tumors at an early stage is an important goal of medical imaging systems, which is very critical for successful treatment of cancer. The clinically accepted imaging tool for breast cancer detection is mammography. However mammography has several disadvantages. Since breast tissue is compressed during imaging, it is very uncomfortable for the patient. It employs ionizing radiation which itself increases the risk of cancer. This method also has high false negative and false positive rates. Therefore, a huge amount of research is going on to develop comfortable, reliable and risk free imaging methods.

Imaging of biological tissues for cancer detection in RF/Microwave frequency spectrum is a hot research topic since malign tumors are reported to have higher conductivity and permittivity values than normal tissue in this frequency range. In microwave imaging systems, high frequency electromagnetic signals are transmitted to tissue using antennas and the backscattered signals are received by receiving antennas. The images are reconstructed revealing dielectric distribution inside the tissue. The microwave imaging systems can be grouped according to the bandwidth: Narrow-band systems and ultra wide band (UWB) systems. In narrow band systems, a large number of antennas are placed around the tissue. Generally one antenna is active at a time while the others are receiving.

Dielectric maps are generated using inverse solution techniques. Narrow band systems produce low resolution images. In UWB systems very short pulses are sent to the tissue and the scattered signals are received by a number of antennas placed around the tissue. These types of systems generate relatively high resolution images. "WO 03003907 A2" document with title "Space-Time Microwave Imaging for Cancer Detection" is an example of this method, which can be considered as the state-of-the art. However, a large scale study on the electrical properties of normal and malign tissues reports that the dielectric contrast between the normal tissue with low adipose content and the malign tissues is low[1]. Hybrid acoustic-electromagnetic methods and contrast enhanced imaging methods are reported to be useful in enhancing the contrast between malign/normal tissues. In thermo-acoustic-electromagnetic imaging approach, the tissue is exposed to microwave signals, and acoustic signals due to temperature changes are acquired by the transducers around the tissue[2]. Thermal maps that depend on the electrical conductivity distribution at that frequency are generated. In order to increase contrast between normal and malignant tissues, injection of contrast-enhancement materials are also reported for this kind of systems [3]. Another type of method for tumor detection is the utilization of radiation force of ultrasound waves. The elasticity property of the tissue is imaged in these methods. It is reported that the Young's constant of malignant tissues are higher than the normal tissues by a factor of 3 to 13[4], which means that the malignant tissues are stiffer than normal tissues. In general ultrasound, acoustic reflection properties of the tissues are imaged. Since the acoustic reflection coefficient of malignant tissues and normal tissues are similar, tumor detection at the early stage is not possible by general ultrasound. However, using the radiation force of focused ultrasound waves, it is possible to generate motion inside the tissue and generate images revealing elasticity distribution inside the tissue.

In one elasticity imaging method for which the ultrasound waves are used, sinusoidal motion is induced inside the tissue at the focus of the ultrasound transducer(s). The motion induces shear waves which are detected by the hydrophones placed on the tissue (Shear wave Imaging). In another similar method, sinusoidal motion is generated and amplitude, frequency and phase of the displacement at the focus are observed by another ultrasound transducer (Harmonic Motion Imaging). The detected signal is processed to get elasticity information of the tissue at the focal point. This method is covered in WO2005002447 A1 patent document.

In mammography, which is the clinically used method for tumor detection, breast is compressed and two dimensional high resolution images are obtained by exposing the tissue to a low dose of ionizing X-ray radiation. Tumor is discriminated from healthy tissue via the contrast of X-ray absorption constant. It is possible that the tumor may be hidden inside a dense healthy tissue such as fibro glandular tissue, which can not be discriminated in the images. In a similar method, which is called tomo-synthesis, 3 dimensional images are formed by scanning the X-ray generator and detector around the tissue. This method is more successful in finding the tumors hidden inside dense tissue. However, ionizing radiation is used as in mammography and patients are advised to avoid this type of imaging unless it is necessary.

In patent document number US 20090316854 A1, a system is described which sends ultrasonic waves to the tissue to move tumor. A single frequency microwave imaging system is utilized. The resolution is increased using an X-Ray device as in mammography. The disadvantages of the mammography, patient discomfort and exposure to ionizing radiation are still present for this multi-modality imaging system.

In another prior art shown in patent document US5099848 A. the breast tissue to be imaged is conformed to a regular geometry such as cylinder. The tissue is vibrated with a low frequency source to excite vibrational eigenmodes. An imaging transceiver system is used to scan the vibrating object and generate elastic constants using vibration amplitudes derived from the Doppler component of the reflected signal. If the imaging transceiver system employs electromagnetic radiation, the received signals will also depend on the electrical properties (i.e. conductivity and permittivity distribution) of the breast tissue. The document does not cover how to detect the presence of a tumor for an electromagnetic interrogator. In addition, conformation of the breast tissue in a geometrical structure is not comfortable for the patient.

In another prior art (US6026173), tissue dielectric properties are imaged using electromagnetic radiation by a plural number of emitter/receivers and multiple microwave frequencies for diagnostic purposes. However, if the dielectric properties of the normal tissue and the diseased tissue are similar, which is the case for a normal high water content tissue and a malignant tumor, the described method will not be able to differentiate the pathologic tissue and the healthy tissue.

Yet in another prior art (US 2009/0281422 A1), focused ultrasound waves are used to generate a focused vibrating region inside the tissue. An electromagnetic transceiver system is used to detect the Doppler component of the reflected signal. It is assumed that the tissue is homogenous, and tissue elasticity contrast is small for benign tissue and tumor. Therefore, the reflected signal is argued to depend only on the electrical properties of the tissue. It is argued that the presence of the Doppler component on the received signal indicates that there is a tumor in the vibrating region. However, the tissue is an inhomogeneous media including high medium, or low adipose content tissues and the electrical reflectivit rLof normal fibro-glandular or connective tissues may be as high as tumors[1]. In addition, elastic constant of normal tissues and tumor was shown to be different by a factor of 3 to 13[4]. In fact, the backscattered signals and generated images depend on both electrical properties and the elastic properties of the tissue. Therefore, there is still a need for a method to detect the presence of a tumor for a realistic scenario in which the tissue is inhomogeneous and the elasticproperties are taken into account.

## Aims for the Development of the Invention

The aims for the development of the invention, hybrid mechanical-electromagnetic imaging method and the system thereof, are:

- Obtaining a high resolution imaging system without using ionizing radiation.

- Obtaining a more reliable imaging system with the use of combined elastic and electrical properties of the tissue,

- Obtaining a non-invasive comfortable imaging system for the patient, in which no compression is required.

## Summary of the Invention

The hybrid mechanical-electromagnetic imaging method and the system thereof which is subject to this patent document, uses single or plural number of focused ultrasonic transducer(s) that send ultrasound waves to the tissue at different radiation force frequencies at different times, which in turn induces local vibrations at different frequencies and different amplitudes. Microwave signals are transmitted to the tissue for each radiation force frequency of ultrasound excitation. The scattered signals which contain a Doppler frequency component because of the motion inside the tissue are received by a microwave receiver. The Doppler frequency component properties (amplitude, frequencies and phases) of the signals are processed for each radiation force frequency. The resultant data reveals the combined elasticity and electrical (dielectric constant and conductivity) properties of the tissue at the focal point. The focal point is scanned throughout the tissue volume and 3D images are generated. Tumors or malignancies can be identified in the images.

## Description of the Figures

The Figures and the related descriptions for the invention, hybrid mechanical-electromagnetic imaging method and the system thereof, described with this document are given below:

Figure-1: Block diagram of the system for hybrid mechanical-electromagnetic imaging method.
Figure-2: Flow chart of the system for hybrid mechanical-electromagnetic imaging method

Figure-3: Detailed block diagram of the system for hybrid mechanical-electromagnetic imaging method
Figure-4: Hybrid mechanical-electromagnetic imaging system
Figure-5: Simulation geometry for tumor detection inside healthy high water content tissue with hybrid mechanical-electromagnetic imaging system
Figure-6: Differential amplitude at the Doppler frequency for 125 Hz and 250 Hz ultrasound excitation frequencies when tumor is present or not inside a high water content tissue
Figure-7: Differential amplitude at the Doppler frequency for 125 Hz and 500 Hz ultrasound excitation frequencies when tumor is present or not inside a high water content tissue

## Description of the Parts and Components of the Invention

The parts and components in the figures for the hybrid mechanical-electromagnetic imaging method and the system using this method, described in this document are numbered for better understanding. The numerals referred to in the following description correspond to the following:

1. Focusing Ultrasound Transducer
2. Object to be imaged
3. Microwave Transmitter
4. Microwave Receiver
5. Data Processing Unit
6. Display Screen
7. Ultrasound driver circuitry
8. Microwave transmitter antenna(s)
9. Microwave receiver antenna(s)
10. Focused region
11. Healthy high-water content tissue
12. Tumor

## Detailed Description of the Invention

The steps of operation for the invention hybrid mechanical-electromagnetic imaging method and the system thereof, described by the claims basically comprise

- Generating motion at a focused region (10) inside the object (2) using focused ultrasound waves by means of feeding a single focused ultrasound transducer (1) (which focus is at the focused region (10)), with amplitude modulated signals of modulation frequency of $f_n$, or feeding two focused transducers (1) (which focuses coincide at the focused region (10)), with slightly different frequency signals with frequency difference $f_n$.,
- Sending microwave signals at center frequency $f_m$, to the focused region (10) which is vibrating.
- Receiving the scattered microwave signal from the object (2), and using microwave components (mixers, filters, couplers, etc.) filtering out the first Doppler component.
- Sampling the Doppler component and processing the received data.
- Repeating the previous steps with various $f_n$ frequency values generated by ultrasound transducer(s) (1) for the same focused region (10).
- Repeating the previous procedure for various microwave operating center frequencies $f_m$ for higher resolution images than the images obtained using single microwave frequencies $f_m$.
- Generating a compound data that depends on both elasticity and electrical properties of the focused region (10), using all the data gathered for this position.
- Scanning the focused region (10) throughout the tissue volume (2) and by means of processing all the gathered data, generating graphics or images.
- Detecting the presence and position of tumor(s) using the graphics or images generated in the previous steps.

A singular or plural number of focused ultrasound transducer (1), microwave transmitting (8) and receiving antennas (9) are placed around or upon the object (2) to be imaged. A signal generator or local oscillator is used for generating the ultrasound signal preferably between 0.5-10 MHz frequency range. This signal is multiplied by another modulating signal which is generated by another local oscillator or signal generator. The frequency of modulating signal is preferably between 0.1 kHz - 10 kHz. The amplitude modulated signal is amplified and fed to the focused ultrasound transducer (1) or transducer array. In another embodiment, two focused ultrasound transducers (1) or transducer arrays which are focused at the same point are fed by two different signal generators with a frequency difference of preferably 0.1 kHz-5 kHz.

A radiation force inside the object (2) is generated by this way. The force can be written as:

$$F = \frac{2\alpha I}{C_s} \qquad\qquad (E1)$$

where $a$ (1/m) is the absorption constant of the tissue, $I$ (W/cm$^2$) is the intensity of the ultrasound beam, $C_s$ (m/s) is the speed of ultrasound in tissue. The short-term time average intensity of the beam can be expressed as:

$$I = \frac{P_0^2}{4\rho c_s}\cos(\Delta\omega t) \qquad\qquad (E2)$$

where $P_0$ is the amplitude of the pressure wave, $\Delta\omega$ is the 2 times modulation frequency, $\omega_u$ is the carrier frequency, $\rho$ is the density of tissue.

Elasticity parameters Young's Modulus (*E*) and Poisson's ratio (*v*) of the tissue is related to displacement of the tissue and the radiation force by:

$$E = \frac{2(1-v)^2 F\, r_b}{X_0 A} \qquad\qquad (E3)$$

where F is the acoustic radiation force given in (E1), $r_b$ is the radius of the beam at the focus, *A* is the cross sectional area of the beam at the focus, $X_0$ is the maximum displacement of the tissue.

The peak value of local displacement $X_0$ at the focused point (10) is determined by the mechanical properties (elasticity/stiffness) of the local tissue as well as the intensity and frequency of the ultrasound.

A microwave signal is generated by a signal generator or local oscillator for which the center frequency is preferably in 0.5 GHz-10 GHz range. A coupler is used to sample this signal to use as local oscillator for down-conversion of the received signal. The transmitter signal is amplified (and band pass filtered if necessary) and fed to the microwave transmitter antenna (8) or antenna array which is coupled to the tissue. The antenna/antenna array is preferably designed specially to couple the microwave signal to the tissue. A coupling medium can also be used for coupling microwave power to the tissue. The microwave signal is scattered from the tissue and received by the microwave receiver antenna (9) or antenna array which is also designed for microwave signal reception from the tissue.

In one embodiment, an interrupted continuous-wave microwave signal is transmitted to the tissue which has a local harmonic motion at the region of the focus (9) of the ultrasound transducer. If the output signal from the transmitter antenna is $S_{TX}(t) = A\cos(\omega_m t)$, the received signal from receiving antenna (9) due to vibrating tumor can be written as:

$$S_{RX}(t) = B[1 + M\sin(\Delta\omega t)]\cos\left(\omega_m t + \frac{4\pi R}{\lambda} + K\sin(\Delta\omega t) + \emptyset\right) \qquad (E4)$$

where R is the distance from antennas to locally vibrating tumor, $\omega_m$ is the microwave operating frequency, $\Delta\omega$ is the vibration frequency of the tissue, M and K are the change in amplitude and phase of the signal respectively, when the tumor is displaced a maximum amount of $X_0$. It is assumed that the focused region (10) which locally moving is on the midplane of microwave transmitting (8) and receiving (9) antennas. $X_0$ is in the order of micrometers and the effect of amplitude modulation (M) can be shown to be very small as compared to the phase modulation (K). Thus, M can be neglected in the analysis. The cosine term in the right hand side of equation (E4) can be expressed as:

$$\cos\left(\omega_m t + \frac{4\pi R}{\lambda} + K\sin(\Delta\omega t) + \emptyset\right) = \cos\left(\omega_m t + \emptyset + \frac{4\pi R}{\lambda}\right)\cdot\cos\left(K\sin(\Delta\omega t)\right)$$

$$-\sin\left(\omega_m t + \emptyset + \frac{4\pi R}{\lambda}\right)\cdot\sin\left(K\sin(\Delta\omega t)\right) \quad (E5)$$

Since K is very small (K<<1), cosine and sine terms resulting from the displacement can be written as:

$$\cos\big(K \sin(\Delta\omega t)\big) \approx 1 \qquad\qquad (E6)$$

$$\sin\big(K \sin(\Delta\omega t)\big) \approx K \sin(\Delta\omega t) \qquad\qquad (E7)$$

Equation (E5)can be written as:

$$\cos\left(\omega_m t + \frac{4\pi R}{\lambda} + K \sin(\Delta\omega t) + \emptyset\right)$$
$$= \cos\left(\omega_m t + \emptyset + \frac{4\pi R}{\lambda}\right) - \sin\left(\omega_m t + \emptyset + \frac{4\pi R}{\lambda}\right) . K \sin(\Delta\omega t)$$
$$(E8)$$

The received signal has a component at the operating ($\omega_m$) frequency and also two different main frequency components at ($\omega_m + \Delta\omega$) and ($\omega_m$ - $\Delta\omega$). The component of the received signal at $\omega_m$ angular frequency may practically be very small compared to clutter and leakage. Since $\Delta\omega$ is known, Doppler filters and other components in the microwave receiver (4) receiving circuitry can be optimized for sensing this frequency component. The bandwidth of the receiver, thus, can be made minimum to increase SNR.

The received signal can be written as:

$$S_{RX}(t) = B\left\{\cos\left(\omega_m t + \emptyset + \frac{4\pi R}{\lambda}\right) + \frac{K}{2}\cos\left(\omega_m t - \Delta\omega t + \frac{4\pi R}{\lambda} + \varphi\right) - \frac{K}{2}\cos\left(\omega_m t + \Delta\omega t + \frac{4\pi R}{\lambda} + \varphi\right)\right\} \qquad (E9)$$

The received signal is amplified (and bandpass filtered) and mixed with the coupled signal from transmitter. In order to decrease phase noise and increase the sensitivity of the receiver, a delay line can be inserted between the coupler and the mixer. The output signal have DC component and a Doppler component due to the local motion of the tissue. Using a Doppler filter the DC component and other unrelated frequency components are removed from the signal. This signal is stored for further signal processing. It may be preferred that the data storing from the microwave receiver (4) is started before the ultrasound signal is on and stopped after the ultrasound transducer (1) is turned off. Monitoring the dynamic behavior of the received signal may also be useful for tumor identification, since the reflected signal characteristic may change because of the induced shear waves.

The frequency of the radiation force of ultrasound is changed and the data is stored for different frequency of radiation force excitation.

The frequency of the microwave can be changed and the data is stored for different frequency of microwave excitation.

The focus of the ultrasound is changed either mechanically or electronically and the data is also stored for other parts of the tissue.

Using all the data, images are generated. Using the generated images, the position and existance of tumor can be found. In one preffered embodiment a line image is formed. The system is moved to another lateral position by the operator and another line image is formed.

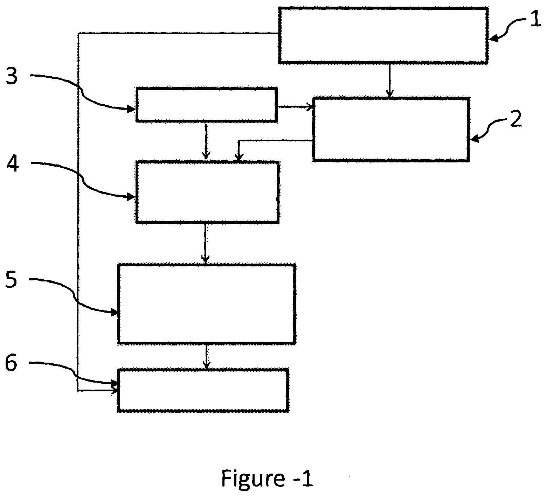

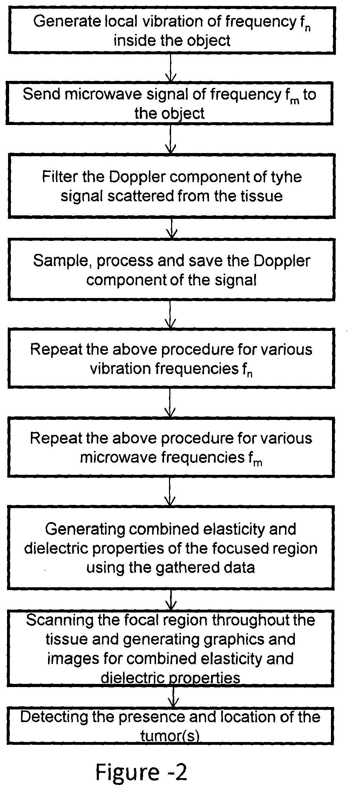

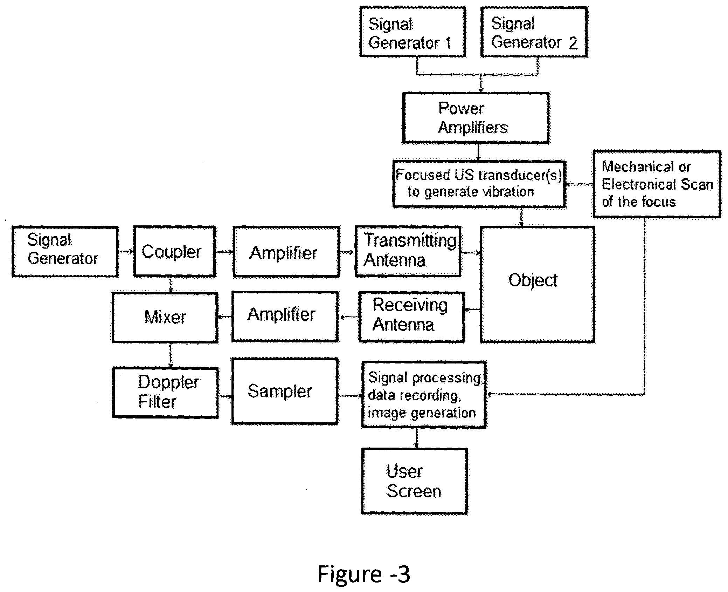

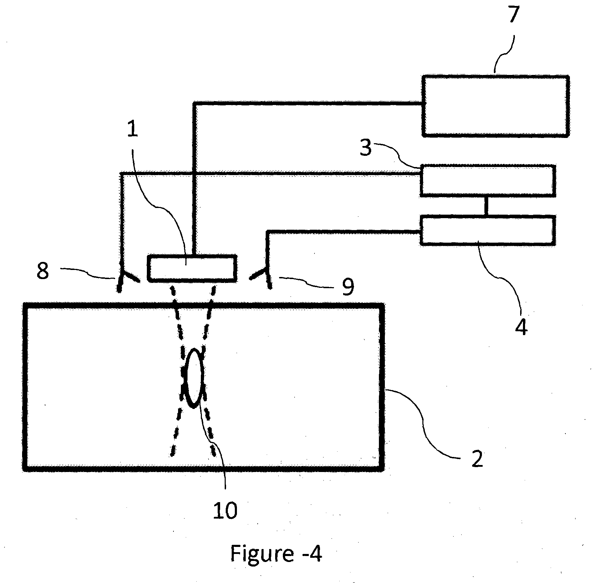

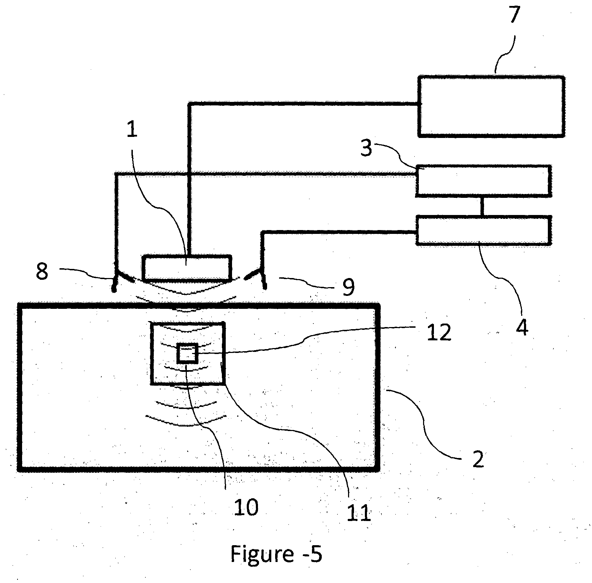

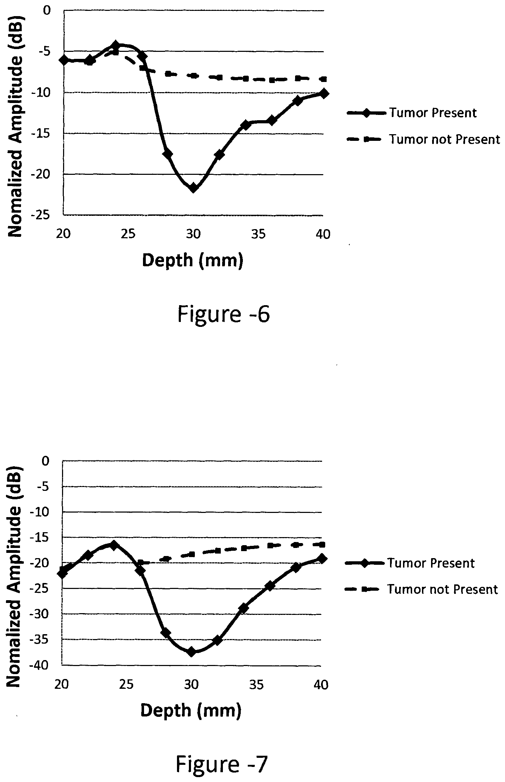

A simulation study is conducted for the received signal amplitude for the cases when the tumor is present or not inside a high water content (fibroglandular) tissue. The electrical parameters (dielectric constant and conductivity) for the normal low water content tissue, high water content (fibroglandular) tissue and the malignant tumor is taken from [1] and elastic parameter ratio of 1:4 for healthy and malignant tumor is assumed. The focused region (10) is scanned from 20 mm depth to 40 mm depth from the skin for various vibration (radiation force) frequencies (125 Hz, 250 Hz and 500 Hz). The output power of ultrasound transducer (1) and microwave transmitter antenna (8) remained constant. The cubic tumor of 3 mm edge length is introduced at 30 mm depth from the skin, inside a cubic fbroglandular tissue of 10mm edge length. The amplitude ratios of the received signals of 125Hz and 250 Hz, and 125 Hz and 500 Hz are plotted in figures 6 and 7 respectively. When the frequency of vibration (radiation force) is increased for the same ultrasound output power, the signal level decreases because of the decrease in displacement amplitude. However, the signal level drops drastically when the tumor is present. It makes a minimum at the position of the tumor. If the tissue is scanned laterally the combination of similar data will result in a dark region in the image where the tumor is present.

It is better to apply different ultrasound radiation force frequencies and observe the changes in the received signal characteristics (amplitude, phase, frequency) for a more reliable system. It should also be noted that the bandwidth of the receiver (4) should be low to decrease the thermal noise of the system. Another limiting factor is that the received signal power level at the Doppler frequency should be greater than the phase noise of the coupled signal from the transmitter at those Doppler frequencies. In the system design, the transmitter power levels of the ultrasound and RF, and also the vibration frequency and RF frequency should be selected carefully in order to acquire reliable data for image formation without harming the tissue,

System using hybrid mechanical-electromagnetic imaging method is mainly composed of

- Focusing Ultrasound Transducer(s) (1) which sends ultrasound waves to the designated region (10),
- Microwave Transmitter (3) that sends microwaves to the vibrating region
- Microwave Receiver (4) which receives reflected microwaves signals from the object to be imaged (2)
- Data processing unit (5) which the Doppler component of the signal is obtained and matched with the related material property of the tissue,
- Display screen (6) which displays the generated image of the object to be imaged (2)
- Ultrasound driver circuitry (7) that generates signals of ultrasounds for the desired frequencies (7)
- Microwave transmitter antenna(s) (8) which sends microwaves to the focused region (10)
- Microwave receiver antenna(s) (9) that receives reflected microwaves,

## References

1. Lazebnik, M., et al., A large-scale study of the ultrawideband microwave dielectric properties of normal, benign and malignant breast tissues obtained from cancer surgeries. Physics in Medicine and Biology, 2007. 52(20): p. 6093-6115.

2. Marshal, A., Booske, İ.H., and Hagness, S.C., Towards contrast-enhanced microwave-induced thermoacoustic imaging of breast cancer: an experimental study of the effects of microbubbles on simple thermoacoustic targets. Physics in Medicine and Biology, 2009. 54(3): p. 641-650.

3. Yao, L., Guo, G.F., and Jiang, H.B., Quantitative microwave-induced thermoacoustic tomography. Medical Physics, 2010. 37(7): p. 3752-3759.

4. Samani, A., Zubovits, J., and Plewes, D., Elastic moduli of normal and pathological human breast tissues: an inversion-technique-based investigation of 169 samples. Physics in Medicine and Biology, 2007. 52(6): p. 1565-1576.

## Claims

1. A high resolution, non-invasive, and hybrid mechanical-electromagnetic tissue imaging method making use of combined elastic and dielectric properties of the tissue, without using ionizing radiation, comprising the steps of

    a. Generating vibration at a focused region (10) inside the tissue of an object (2) using focused ultrasound waves by means of feeding a single focused ultrasound transducer (1) with amplitude modulated signals of modulation frequency $f_n$ focused at the focused region, or feeding two focused ultrasound transducers (1), with slightly different frequency signals with frequency difference $f_n$, with foci coinciding at the same focused region, to induce vibration at the focused region with a vibration frequency ($f_n$);
    b. Sending microwave signals at center frequency $f_m$, to the focused region (10) which is vibrating;
    c. Receiving scattered microwave signal from the object (2), and, using microwave components, filtering out a Doppler component;
    d. Sampling, processing and saving the Doppler component of the received scattered microwave signal; **characterized by**
    e. Repeating the previous steps with various $f_n$ vibration frequencies ($f_n$);
    f. Generating a compound data that depends on both elasticity and dielectric properties of the focused region (10), using all the Doppler components gathered for this focused region,
    g. Scanning the focused region (10) throughout the tissue of the object to be imaged (2) and by means of processing all the compound data, generating graphics or images.

2. Hybrid mechanical-electromagnetic imaging method according to claim 1, in which the steps a, b, c, d, e, f of the hybrid mechanical-electromagnetic imaging method are repeated, for various microwave center frequencies $f_m$, for obtaining a higher resolution image as compared to an image obtained using a single microwave frequency $f_m$.

3. Hybrid mechanical-electromagnetic tissue imaging system configured to perform the method of claim 1.

**Patentansprüche**

1. Ein Hybrid-mechanisch-elektromagnetische Gewebebildgebungsverfahren, die nicht invasive ist, die einer hohen Auflösung hat, ohne ionisierender Strahlung unter Verwendung der elastischen und dielektrischen Eigenschaften, die folgende Schritte umfasst:

   a. Modulationsfrequenz ($f_n$) fokussiert Bereich, einen einzigen fokussierten Ultraschallwandlerhalter (1) auf die von der Amplitude eines Objekts unter Verwendung von fokussierten Ultraschallwellen, die durch Einspeisen (2) modulierte Signale fokussiert, der Fokussierung in dem Gewebe (10) Schwingungserzeugung oder Schwingungsfrequenz ($f_n$) mit Schwingungen im Fokus der konfokalen zusammenfällt, mit dem Fokus auf die Unterschiede in der Frequenz ($f_n$) hat eine etwas andere Frequenzsignal mit zwei fokussierten Ultraschallwandlerhalter (1) eine Ernährung zu beginnen;
   b. das Mikrowellensignal, von der Mittenfrequenz ($f_m$) zum orientierten Bereich (10) zu senden,
   c. von einem Objekt (2), die gestreute Mikrowellensignale einzunehmen, damit die Mikrowellen-Komponenten zu verwenden, um Doppler-Komponenten zu filtern;
   d. die Beprobung der empfangenen gestreuten Mikrowellensignal Dopplerkomponente , zu verarbeiten und aufzuzeichnen

   wird **dadurch gekennzeichnet, dass**:

   e. mit verschiedenen Schwingungsfrequenzen ($f_n$) die vorherigen Schritte wiederholen;
   f. Durch die Verwendung der Doppler-Komponenten für alle betroffenen Regionen, auf dem Bereich konzentriert (10), die verbunden ist, sowohl für die Erzeugung der Datenverbund dielektrischen Eigenschaften Elastizität zu erzeugen;
   g. Anzeige (2) durch das Gewebe des Objekts, durch die Verarbeitung von Daten und Grafiken oder Bilder aller Verbindungen den Fokusbereich (10) abzutasten.

2. Hybrid-mechanisch-elektromagnetischen Abbildungsverfahren nach Anspruch 1, **dadurch gekennzeichnet, dass** hybrider mechanische und elektromechanische Abbildungsverfahren, im Vergleich zu Verwendung einer einzige Mikrowellenfrequenz ($f_m$) für die Schritte a b, c, d, e, f mit dem Mikrowellenmittenfrequenz eine bessere Auflösung erhält und wiederholt.

3. Ein Hybrid mechanisch-elektromagnetische Gewebebildgebungssystem konfiguriert ist, das Verfahren nach Anspruch 1 durchzuführen.

**Revendications**

1. Méthode d'imagerie de tissu hybride mécanique électromagnétique, à haute résolution et non invasive, utilisant des propriétés élastiques et diélectriques combinées du tissu, sans utiliser de rayonnement ionisant, comprenant les étapes consistant à:

   a. Générer de vibrations au niveau d'une zone focalisée (10) à l'intérieur de tissu d'un objet (2) en utilisant des ondes ultrasonores focalisées au moyen d'alimentation d'un seul transducteur ultrasonore focalisé (1) avec des signaux modulés en amplitude de fréquence de modulation de ($f_n$) focalisés dans la région focalisée ou d' alimentation de deux des transducteurs à ultrasons focalisés (1), avec des signaux de fréquence légèrement différents avec une différence de fréquence (fn) avec des foyers coïncidant dans la même région focalisée, ($f_n$) pour induire une vibration dans la région focalisée avec une fréquence de vibration ($f_n$);
   b. Envoye des signaux micro-ondes à la fréquence centrale ($f_m$), à la région focalisée (10) qui vibre.

c. Récevoir le signal micro-onde diffusé par l'objet (2), et, utilizer composants micro-ondes, filtrer un composant Doppler;

d. Échantillonner, traiter et enregistrer le composant Doppler du signal micro-ondes diffusé reçu; **caractérisé par**

e. Répéter les étapes précédentes avec diverses fréquences de vibration ($f_n$);

f. Générer une donnée composée qui dépend à la fois de l'élasticité et des propriétés diélectriques de la région focalisée (10), en utilisant tous les composants Doppler rassemblés pour cette région focalisée.

g. Balayer la région focalisée (10) à travers le tissu de l'objet à imager (2) et au moyen de traitement de toutes les données composées, générer des graphiques ou des images.

2. Procédé d'imagerie mécanique- électromagnétique hybride selon la revendication 1, dans lequel les étapes a, b, c, d, e, f de la méthode d'imagerie mécanique - électromagnétique hybride sont répétées pour différentes fréquences centrales micro-ondes ($f_m$) pour obtenir une image de résolution plus élevée comparée à une image obtenue en utilisant une fréquence micro-ondes unique.

3. Système d'imagerie mécanique électromagnétique hybride de tissu configuré pour exécuter le procédé de la revendication 1.

EP 2 908 716 B1

Figure -1

10

```
┌─────────────────────────────────────────┐
│   Generate local vibration of frequency fₙ │
│             inside the object             │
└─────────────────────────────────────────┘
                     ↓
┌─────────────────────────────────────────┐
│  Send microwave signal of frequency fₘ to │
│                the object                 │
└─────────────────────────────────────────┘
                     ↓
┌─────────────────────────────────────────┐
│   Filter the Doppler component of tyhe    │
│     signal scattered from the tissue      │
└─────────────────────────────────────────┘
                     ↓
┌─────────────────────────────────────────┐
│    Sample, process and save the Doppler   │
│          component of the signal          │
└─────────────────────────────────────────┘
                     ↓
┌─────────────────────────────────────────┐
│    Repeat the above procedure for various │
│          vibration frequencies fₙ         │
└─────────────────────────────────────────┘
                     ↓
┌─────────────────────────────────────────┐
│    Repeat the above procedure for various │
│          microwave frequencies fₘ         │
└─────────────────────────────────────────┘
                     ↓
┌─────────────────────────────────────────┐
│       Generating combined elasticity and  │
│  dielectric properties of the focused region │
│           using the gathered data         │
└─────────────────────────────────────────┘
                     ↓
┌─────────────────────────────────────────┐
│ Scanning the focal region throughout the  │
│    tissue and generating graphics and     │
│    images for combined elasticity and     │
│            dielectric properties          │
└─────────────────────────────────────────┘
                     ↓
┌─────────────────────────────────────────┐
│ Detecting the presence and location of the │
│                 tumor(s)                  │
└─────────────────────────────────────────┘
```

Figure -2

```
┌─────────────┐  ┌─────────────┐
│ Signal      │  │ Signal      │
│ Generator 1 │  │ Generator 2 │
└─────────────┘  └─────────────┘
        │                │
        └────────┬───────┘
                 ▼
        ┌─────────────────┐
        │     Power       │
        │   Amplifiers    │
        └─────────────────┘
                 │
                 ▼
   ┌──────────────────────────┐      ┌──────────────────┐
   │ Focused US transducer(s) │◄─────│  Mechanical or   │
   │  to generate vibration   │      │ Electronical Scan│
   └──────────────────────────┘      │   of the focus   │
                 │                    └──────────────────┘
                 ▼
┌───────────┐  ┌─────────┐  ┌───────────┐  ┌──────────────┐  ┌──────────┐
│  Signal   │─►│ Coupler │─►│ Amplifier │─►│ Transmitting │─►│          │
│ Generator │  └─────────┘  └───────────┘  │   Antenna    │  │  Object  │
└───────────┘       │                      └──────────────┘  │          │
                    ▼                                         └──────────┘
               ┌─────────┐  ┌───────────┐  ┌──────────────┐      │
               │  Mixer  │◄─│ Amplifier │◄─│  Receiving   │◄─────┘
               └─────────┘  └───────────┘  │   Antenna    │
                    │                      └──────────────┘
                    ▼
               ┌─────────┐  ┌─────────┐  ┌──────────────────┐
               │ Doppler │─►│ Sampler │─►│ Signal processing│◄──────
               │ Filter  │  └─────────┘  │  data recording, │
               └─────────┘               │ image generation │
                                         └──────────────────┘
                                                  │
                                                  ▼
                                         ┌──────────────┐
                                         │     User     │
                                         │    Screen    │
                                         └──────────────┘
```

Figure -3

Figure -4

Figure -5

Figure -6

Figure -7

## REFERENCES CITED IN THE DESCRIPTION

**Patent documents cited in the description**

- WO 03003907 A2
- WO 2005002447 A1
- US 20090316854 A1
- US 5099848 A
- US 6026173 A
- US 20090281422 A1

**Non-patent literature cited in the description**

- **LAZEBNIK, M. et al.** A large-scale study of the ultrawideband microwave dielectric properties of normal, benign and malignant breast tissues obtained from cancer surgeries. *Physics in Medicine and Biology,* 2007, vol. 52 (20), 6093-6115
- **MARSHAL, A. ; BOOSKE, İ.H. ; HAGNESS, S.C.** Towards contrast-enhanced microwave-induced thermoacoustic imaging of breast cancer: an experimental study of the effects of microbubbles on simple thermoacoustic targets. *Physics in Medicine and Biology,* 2009, vol. 54 (3), 641-650
- **YAO, L. ; GUO, G.F. ; JIANG, H.B.** Quantitative microwave-induced thermoacoustic tomography. *Medical Physics,* 2010, vol. 37 (7), 3752-3759
- **SAMANI, A. ; ZUBOVITS, J. ; PLEWES, D.** Elastic moduli of normal and pathological human breast tissues: an inversion-technique-based investigation of 169 samples. *Physics in Medicine and Biology,* 2007, vol. 52 (6), 1565-1576